# EUROPEAN PATENT SPECIFICATION

(11) **EP 4 019 133 B1**
(45) Date of publication and mention of the grant of the patent: **21.02.2024**
(21) Application number: 21210091.1
(22) Date of filing: 24.11.2021
(51) Int. Cl.: B01L 3/00, B01L 7/00, C12Q 1/68

(54) **MICROFLUIDIC DEVICE AND NUCLEIC ACID AMPLIFICATION METHOD**
MIKROFLUIDISCHE VORRICHTUNG UND NUKLEINSÄUREAMPLIFIKATIONSVERFAHREN
DISPOSITIF MICROFLUIDIQUE ET PROCÉDÉ D'AMPLIFICATION D'ACIDE NUCLÉIQUE

(30) Priority: 22.12.2020 JP 2020212728
(43) Date of publication of application: 29.06.2022
(73) Proprietor: Funai Electric Co., Ltd., Daito, Osaka, 574-0013 (JP)
(72) Inventor: TANABE, Hideki, Osaka, 574-0013 (JP)
(74) Representative: Becker, Eberhard

(56) References cited:
- US-B1- 7 235 406

## Description

### BACKGROUND

### [Technical Field]

The disclosure relates to a microfluidic device and a nucleic acid amplification method.

### [Description of Related Art]

Polymerase chain reaction (PCR) is one of the methods for amplifying nucleic acids. PCR is a method in which a fragment of DNA specified as a test target is mixed with a suitable primer and a specific chemical such as an enzyme, and a thermal cycle including heating and cooling is repeated in a PCR tester to amplify a specific region of the target DNA sample. Recently, there have also been methods called quantitative PCR (qPCR) and real-time PCR, which analyze the reaction in the process of exponentially increasing DNA by directly observing fluorescence. Such devices and methods for PCR are disclosed, for example, in Patent Document 1: JP 2000-511435 A, Patent Document 2: JP 2018-512882 A, and Patent Document 3: JP 2017-510796 A.

### Related Art

US 7 235 406 B1 discloses a method and device for simultaneously testing a sample for the presence, absence, and/or amounts of one or more a plurality of selected analytes. The device includes a substrate which defines a sample-distribution network having (i) a sample inlet, (ii) one or more detection chambers, and (iii) channel means providing a dead-end fluid connection between each of the chambers and the inlet. Each chamber may include an analyte-specific reagent effective to react with a selected analyte that may be present in the sample, and detection means for detecting the signal.

### SUMMARY

### Problems to be Solved

However, according to the Patent Documents 1-3, in qPCR, when the starting material is defective, for example, when the starting material contains less DNA fragment as the test target than the expected value, compared to the case in which the starting material is as expected, the fluorescence intensity with respect to DNA amplification is weakened, the desired result cannot be obtained, and there is adverse effect on the result. The adverse effect includes, for example, a problem that the amount of DNA fragment that should have been obtained cannot be obtained.

In addition to such a quantitative defect of DNA fragment as the test target, the defect of the starting material may also cause a qualitative defect. The qualitative defect includes, for example, a case in which a DNA fragment at a site different from the assumed DNA fragment is included. Similarly, in this case, there is a problem that a DNA fragment that should have been obtained cannot be obtained.

In view of the above problems, it is an objective of the disclosure to provide a microfluidic device and a nucleic acid amplification method that reduce adverse effects when a starting material is defective.

### Means for Solving the Problems

In order to achieve the above objectives, the present invention provides a microfluidic device as set out in the appended set of claims. The following serves a better understanding of the present invention. According to the disclosure, the microfluidic device a microfluidic device for amplifying a nucleic acid and includes a cartridge (1) storing a solution (30) containing the nucleic acid, and a control part (14) controlling the cartridge. The cartridge (1) includes a tank part (15) storing the solution (30), and a plurality of first chambers (21) holding the solution (30) from the tank part (15). The control part (14) is configured to: control execution of a thermal cycle which changes a temperature of the first chambers (21); count a number of repetitions of the thermal cycle for each of the first chambers and store a count value; acquire a fluorescence intensity of each of the first chambers for each thermal cycle; determine whether the acquired fluorescence intensity of each of the first chambers (21) is within a predetermined range defined in advance for each number of repetitions; determine the first chamber (21) to be a defective chamber when it is determined that the fluorescence intensity is not within the predetermined range; and reset the count value of the defective chamber, discharge the solution from the defective chamber, and fill the defective chamber with a new solution from the tank part.

Accordingly, it is possible to reduce the adverse effect when a quantitative defect or a qualitative defect is present in the starting material contained in the solution held in the first chamber (21). In other words, if a quantitative defect or a qualitative defect is present in the starting material, the control part (14) determines the defective chamber and replaces the solution of the defective chamber with the new solution. That is, the first chamber (21) in which a quantitative defect or a qualitative defect is present in the starting material starts a new thermal cycle with the new solution. Therefore, among the plurality of first chambers (21), the first chambers that have adverse effect can be eliminated or reduced.

The cartridge (1) includes a plurality of first heaters (24) corresponding to the first chambers (21).

Accordingly, since one first heater (24) corresponds to one first chamber (21), in the thermal cycle, the time required for heating the first chamber (21) to the target temperature can be shortened, and the total time required for the thermal cycle can be shortened.

The cartridge (1) includes a plurality of flow path parts (20) connecting the tank part (15) and the first chambers (21). Each of the first chambers (21) includes a first opening (21b) connected to the flow path part (20), and a second opening (21a) for discharging the solution held in the first chamber (21). The control part (14) may generate a bubble in the solution in the defective chamber by the first heater (24) corresponding to the defective chamber, discharge the solution of the defective chamber from the second opening by generation of the bubble, and fill the new solution to the defective chamber from the tank part (15) via the flow path part (20) and the first opening (21a).

Accordingly, the set of the first heater (24) and the first chamber (21) may serve both of the following two functions. One is to heat the solution of the corresponding first chamber (21) in the thermal cycle. The other is to discharge the solution of the defective chamber and fill the defective chamber with the new solution.

For example, the cartridge (1) may include a plurality of first temperature sensors (25) corresponding to the first chambers (21). The control part (14) may acquire data indicating a temperature from the first temperature sensors (25) in the thermal cycle and manage a temperature of each of the first chambers (21).

Accordingly, the speed and accuracy of temperature control in the thermal cycle can be improved for each of the first chambers (21).

For example, the cartridge (1) may include a thermal head part (16). The thermal head part (16) may include a semiconductor substrate (17) and a head film (18) bonded to the semiconductor substrate (17). The semiconductor substrate (17) may include the first heater (24) and the flow path part (20). The first chamber (21) and the flow path part (20) may be formed as a gap formed by bonding the semiconductor substrate (17) and the head film (18) together.

Accordingly, since the cartridge (1) has the same configuration as a cartridge of a thermal inkjet printer, the manufacturing cost can be reduced and the solution can be easily discharged and filled in the first chamber (21) at the order of picolitre to microliter.

For example, the semiconductor substrate (17) may include a substrate heater (23).

Accordingly, since the first chambers (21) may also be heated from the periphery by heating the semiconductor substrate (17), in the thermal cycle, the time required for heating the solution to the target temperature can be shortened, and the total time required for the thermal cycle can be shortened.

For example, the cartridge (1) may include a plurality of second chambers (22), and a plurality of second heaters (26) corresponding to the second chambers (22). Each of the second chambers (22) may be located in the middle of the flow path part (20) and provided at a position between the first opening (21a) of the first chamber (21) and a connection portion between the tank part (15) and the flow path part (20).

Accordingly, since the second chambers (22) are subjected to the thermal cycle as the first chambers (21), the amount of solution subjected to the thermal cycle can be increased, for example, by two times or more.

For example, the control part (14) may discharge, via the defective chamber, the solution of the second chamber (22) that is connected to the defective chamber via the flow path part (20), and fill the second chamber (22) with the new solution from the tank part (15).

Accordingly, since the second chamber (22) connected to the defective chamber also starts a new thermal cycle with the new solution, for example, a volume of the second chamber (22) may be larger than a volume of the first chamber (21).

Accordingly, the amounts of the solution to be processed and as a result of the process can be greatly increased.

For example, the cartridge (1) may include a plurality of second temperature sensors (27) corresponding to the second chambers (22). The control part (14) may acquire data indicating a temperature from the second temperature sensors (27) in the thermal cycle and manage a temperature of each of the second chambers (27).

Accordingly, the speed and accuracy of temperature control in the thermal cycle can be improved for each of the second chambers (22).

For example, the cartridge (1) may include a heat dissipation fin (34) for cooling the thermal head part (16).

Accordingly, the time required for cooling the first chamber (21) to the target temperature in the thermal cycle can be shortened, the total time required for the thermal cycle can be further shortened.

For example, the cartridge (1) may include a fan (37) for cooling the heat dissipation fin (34).

Accordingly, the cooling efficiency can be improved, and as a result, the total time required for the thermal cycle can be further shortened.

For example, the cartridge (1) may include an electronic cooling element (35) for cooling the heat dissipation fin (34).

Accordingly, the cooling efficiency can be improved, and as a result, the total time required for the thermal cycle can be further shortened.

For example, the cartridge (1) may include a needle-shaped heat conductive member (36) that is inserted at an interface between the tank part (15) and the thermal head part (16) to conduct heat to the heat dissipation fin (34).

Accordingly, the cooling efficiency can be further improved, and as a result, the total time required for the thermal cycle can be greatly shortened.

For example, a cap (10) covering the first chambers (21) may be further included.

Accordingly, it is possible to prevent or suppress natural drying of the solution from the openings of the first chambers (21).

For example, a waste liquid tray (11) receiving the solution discharged from the defective chamber may be further included.

Accordingly, the solution discharged from the defective chamber can be appropriately discarded.

For example, a plate having a plurality of wells may be further included to receive the solution discharged from the first chambers (21) excluding the defective chamber, after completion of a predetermined number of thermal cycles.

Accordingly, the solution of the first chambers (21) which are not defective among the plurality of first chambers (21) can be dispensed into the wells of the plate.

For example, a movement control part moving the cartridge (1) at least one-dimensionally may be further included.

Accordingly, the cartridge (1) can be freely moved. For example, the cartridge (1) can be moved to each of the positions of the cap (10), the waste liquid tray (11), the plate (12), the light source (5), the light receiving sensor (6), etc., which are arranged one-dimensionally.

Further, a nucleic acid amplification method according to an aspect of the disclosure is a nucleic acid amplification method in a microfluidic device, the microfluidic device (100) including a cartridge (1) storing a solution (30) containing a nucleic acid, and a control part (14) controlling the cartridge. The cartridge (1) includes a tank part (15) storing the solution (30), and a plurality of first chambers (21) holding the solution (30) from the tank part (15). In the nucleic acid amplification method, a thermal cycle which changes a temperature of the first chambers (21) is controlled. A number of repetitions of the thermal cycle is counted for each of the first chambers and a count value is stored. A fluorescence intensity of each of the first chambers is acquired for each thermal cycle. It is determined whether the acquired fluorescence intensity of each of the first chambers (21) is within a predetermined range defined in advance for each number of repetitions. The first chamber (21) is determined to be a defective chamber when it is determined that the fluorescence intensity is not within the predetermined range. The count value of the defective chamber is reset, the solution is discharged from the defective chamber, and the defective chamber is filled with a new solution from the tank part.

Accordingly, it is possible to reduce the adverse effect when a quantitative defect or a qualitative defect is present in the starting material contained in the solution held in the first chamber (21). In other words, if a quantitative defect or a qualitative defect is present in the starting material, the control part (14) determines the defective chamber and replaces the solution of the defective chamber with the new solution. That is, the first chamber (21) in which a quantitative defect or a qualitative defect is present in the starting material starts a new thermal cycle with the new solution. Therefore, among the plurality of first chambers (21), the first chambers that have adverse effect can be eliminated or reduced.

It is noted that the reference numerals in parentheses in the above description describe the corresponding components in the embodiment for reference only to facilitate understanding, and the above components are not limited to the referenced components of the embodiment.

In addition, the generally comprehensive or specific embodiments may be realized as systems, methods, integrated circuits, computer programs, recording media such as computer-readable CD-ROMs, etc., or may be realized as any combination of systems, methods, integrated circuits, computer programs, and recording media.

### Effects

According to the microfluidic device and the nucleic acid amplification method of the disclosure, it is possible to reduce adverse effects in the presence of a quantitative defect or a qualitative defect in the starting material.

### BRIEF DESCRIPTION OF THE DRAWINGS

FIG. 1 is a block diagram showing a configuration example of a microfluidic device according to the embodiment.
FIG. 2 is a view showing a configuration example of a cross section and a lower surface of a cartridge according to the embodiment.
FIG. 3 is a view showing a dimension example of the cartridge according to the embodiment.
FIG. 4 is a view showing a configuration of a cross section and a lower surface of the cartridge according to a first modification example of the embodiment.
FIG. 5 is a block diagram showing a circuit configuration example of the cartridge according to the embodiment.
FIG. 6 is a circuit diagram showing a configuration example of the first heater and its periphery according to the embodiment.
FIG. 7 is a time chart showing an operation example of heating the solution of the first chamber according to the embodiment.
FIG. 8 is a time chart showing an operation example of discharging the solution of the first chamber according to the embodiment.
FIG. 9 is a view showing positions of moving destinations of the cartridge according to the embodiment.
FIG. 10 is a flowchart showing a unit thermal cycle process in FIG. 11.
FIG. 11 is a flowchart showing an operation for nucleic acid amplification using the microfluidic device according to the embodiment.
FIG. 12 is a flowchart showing a continuation of the operation of FIG. 11.
FIG. 13 is a view showing a cross-sectional configuration example of the cartridge according to a second modification example of the embodiment.
FIG. 14 is a view showing a cross-sectional configuration example of the cartridge according to a third modification example of the embodiment.
FIG. 15 is a view showing a cross-sectional configuration example of the cartridge according to a fourth modification example of the embodiment.
FIG. 16 is a view showing a configuration example of a cross section and a lower surface of the cartridge according to a fifth modification example of the embodiment.

### DESCRIPTION OF THE EMBODIMENTS

Hereinafter, embodiments of the disclosure will be described in detail with reference to the drawings. The embodiments described below all show comprehensive or specific examples. Numerical values, shapes, materials, components, arrangement positions and connections of the components, steps, the sequence of steps, etc. shown in the following embodiments are merely an example and are not intended to limit the disclosure. Further, among the components in the following embodiments, the components not described in the independent claims will be described as arbitrary components. Each figure does not necessarily show each dimension or each dimension ratio and the like in an exact manner.

### (Embodiment)

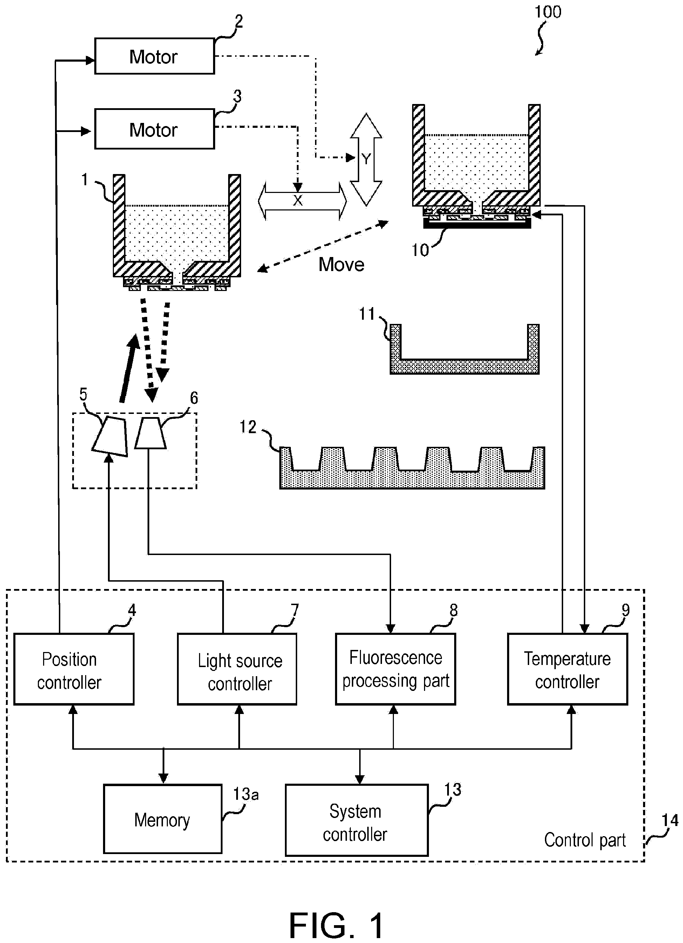

FIG. 1 is a block diagram showing a configuration example of a microfluidic device 100 according to the embodiment. The microfluidic device 100 in the figure includes a cartridge 1, a motor 2, a motor 3, a position controller 4, a light source 5, a light receiving sensor 6, a light source controller 7, a fluorescence processing part 8, a temperature controller 9, a cap 10, a waste liquid tray 11, a plate 12, a system controller 13, and a control part 14. In the figure, two cartridges 1 are illustrated to show that the cartridge 1 is movable, and it does not mean that two cartridges 1 are present.

The cartridge 1 is a container storing a solution 30 in which a DNA fragment defined as a PCR test target is mixed with a suitable primer and a specific chemical such as an enzyme. The cartridge 1 is configured to be movable at least one-dimensionally (in the X-axis direction). It is movable relative to the mounted plate 12 in the X-Y-axis directions.

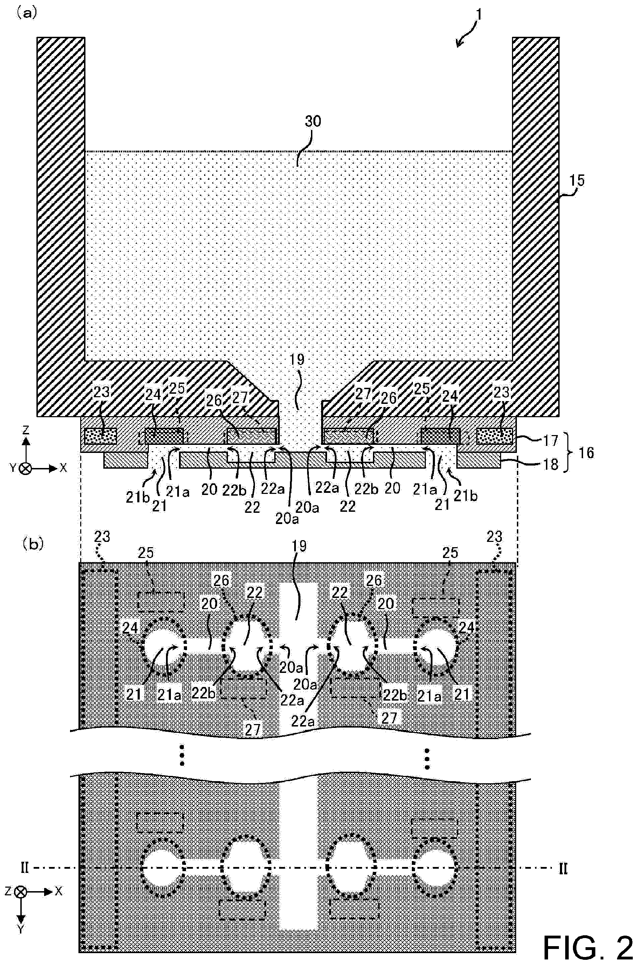

Herein, the configuration of the cartridge 1 will be described with reference to FIG. 2.

FIG. 2 is a view showing a configuration example of a cross section and a lower surface of the cartridge 1 according to the embodiment. (a) of the figure is a cross section of the cartridge 1 cut along the X-Z plane, and shows a cross section along line II-II shown in a dot-dash line in (b) of the figure. (b) of the figure shows a cross section cut along the X-Y plane across a flow path part 20 of (a) of the figure.

As shown in FIG. 2, the cartridge 1 includes a tank part 15 and a thermal head part 16.

The tank part 15 has a tank chamber storing the solution 30, and has an opening part 19 connected to the thermal head part 16 on the bottom surface of the tank part 15. The opening part 19 has a strip-shaped elongated rectangular shape extending along the Y axis.

The thermal head part 16 has a semiconductor substrate 17 and a head film 18 bonded to the semiconductor substrate 17. The flow path part 20, a first chamber 21, and a second chamber 22 are formed by a gap formed by bonding the semiconductor substrate 17 and the head film 18 together. The head film 18 may have a property of transmitting irradiation light of the light source 5 and fluorescence of the second chamber 22. In this case, the fluorescence intensity of the second chamber 22 may also be measured.

The flow path part 20 connects the opening part 19 of the tank part 15 and the first chamber 21. One end of the flow path part 20 is a third opening 20a connected to the opening part 19. Another end of the flow path part 20 is connected to a first opening 21a of the first chamber 21. Further, in the middle of the flow path part 20, the second chamber 22 is provided by enlarging the cross-sectional area of the flow path.

The first chamber 21 holds the solution 30 that flows in from the tank part 15 via the opening part 19 and the flow path part 20. The first chamber 21 has a first opening 21a connected to the flow path part 20 and a second opening 21b exposed on the lower surface. The second opening 21b is also a discharge nozzle.

The second chamber 22 holds the solution 30 flowing in from the tank part 15 via the opening part 19. The second chamber 22 is provided in the middle of the flow path part 20 and also functions as a flow path for carrying the solution 30 to the first chamber 21. The second chamber 22 has a fourth opening 22a through which the solution 30 flows in from the tank part 15, and a fifth opening 22b through which the solution 30 flows out to the first chamber 21 side. The total number of the first chambers 21 included in the cartridge 1 depends on the size of the cartridge 1, but may be, for example, about 32 to 640.

The semiconductor substrate 17 includes a substrate heater 23, a first heater 24, a first temperature sensor 25, a second heater 26, and a second temperature sensor 27.

The substrate heater 23 heats the semiconductor substrate 17 under the control of the temperature controller 9. In the example of the figure, the substrate heater 23 is a strip-shaped elongated resistor arranged on two sides of the semiconductor substrate 17 along the Y direction.

The first heater 24 is provided adjacent to the first chamber 21 in the Z direction and heats the solution 30 of the first chamber 21 under the control of the temperature controller 9.

The first temperature sensor 25 is arranged adjacent to or immediately proximate the first chamber 21 in the -Y direction to detect the temperature of the solution 30 held in the first chamber 21 and notify it to the temperature controller 9.

The second heater 26 is provided adjacent to the second chamber 22 in the Z direction and heats the solution 30 of the second chamber 22 under the control of the temperature controller 9.

The second temperature sensor 27 is arranged adjacent to or immediately proximate the second chamber 22 in the Y direction to detect the temperature of the solution 30 held in the second chamber 22 and notify it to the temperature controller 9.

Next, returning to FIG. 1, the configuration of the cartridge 1 will be described.

The motor 2 moves the cartridge 1 in the Y direction.

The motor 3 moves the cartridge 1 in the X direction.

The position controller 4 controls the motor 2 and the motor 3 to move the cartridge 1 to arbitrary positions in the X direction and the Y direction.

The light source 5 irradiates light to the first chamber 21 or the second chamber 22. The peak wavelength of the light source is, for example, 488 nm. The solution 30 may be stained in advance with a fluorescent substance so as to fluoresce when irradiated with light. The wavelength of the fluorescence is, for example, 522 nm.

The light receiving sensor 6 measures the fluorescence intensity of the first chamber 21 or the second chamber 22 and notifies it to the fluorescence processing part 8. The light source 5 and the light receiving sensor 6 are formed as one unit and are movable relative to the cartridge 1.

The light source controller 7 controls the light source 5 to irradiate the first chamber 21 or the second chamber 22 for each thermal cycle. It performs control of measuring the fluorescence intensity.

The fluorescence processing part 8 performs control of measuring the fluorescence intensity of the first chamber 21 or the second chamber 22 for each thermal cycle.

In the thermal cycle, the temperature controller 9 sequentially performs, for example, steps in stages of (a) denaturation reaction, (b) annealing, and (c) extension reaction. The temperature controller 9 heats the first chamber 21 by heating of the heater in stages (a) and (c). In the transition from (a) to (b), heat dissipation is performed by natural heat dissipation or attaching a heat dissipation material to the cartridge 1. Alternatively, forced cooling is performed by an electronic cooling element or air blowing. The denaturation reaction of (a) is continued at 95°C for 1 minute. The annealing of (b) is continued at 40°C to 55°C for 1 minute. The extension reaction of (c) is continued at 72°C for 1 minute. The time and temperature for continuing each stage of (a), (b) and (c) may be appropriately set depending on the target nucleic acid, and the concentration and type of the solution.

The cap 10 prevents or suppresses the solution 30 held in the first chamber 21 from evaporating from the second opening 21b and drying out.

The waste liquid tray 11 receives the solution 30 discharged from the first chamber 21 having defective fluorescence, as a result of the fluorescence intensity measurement for each thermal cycle.

The plate 12 receives the solution of which the thermal cycle has been completed for a predetermined number of times and the amplification of the DNA fragment has reached the plateau region. The plate 12 has a plurality of wells.

The system controller 13 controls the position controller 4, the light source controller 7, the fluorescence processing part 8, and the temperature controller 9.

A memory 13a stores a program executed by a CPU serving as the system controller 13 and various data. As the various data, the memory 13a may store, for example, a predetermined reference range for each number of thermal cycle repetitions, a count value which is the number of individual thermal cycle repetitions for the first chamber 21, a fluorescence intensity corresponding to the individual count value of the first chamber 21, and values of NG_flag indicating whether the individual count value and fluorescence intensity of the first chamber 21 is defective.

The control part 14 includes the position controller 4, the light source controller 7, the fluorescence processing part 8, the temperature controller 9, and the memory 13a.

Next, dimension examples of the flow path part 20, the first chamber 21, and the second chamber 22 of the cartridge 1 will be described.

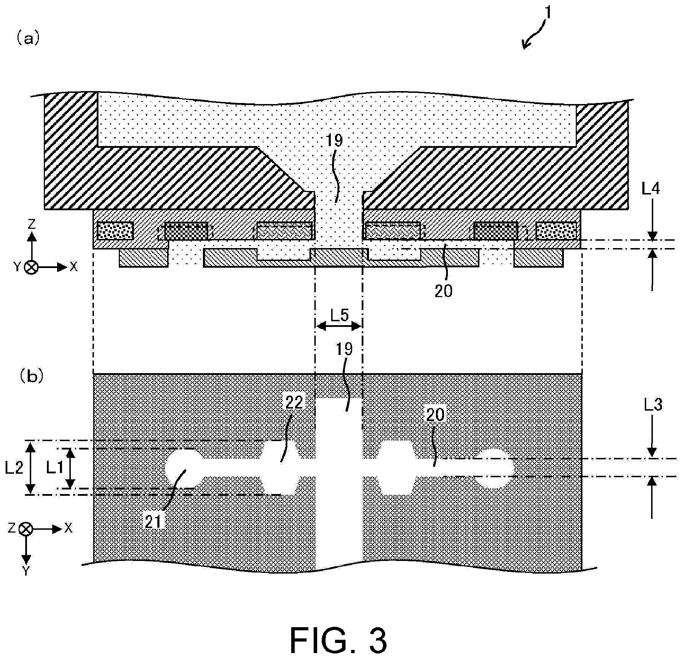

FIG. 3 is a view showing a dimension example of the cartridge 1 according to the embodiment. FIG. 3 shows only a main part of FIG. 2.

A dimension L1 in FIG. 3 indicates the diameter of the first chamber 21 which is circular in a plan view viewed in the Z direction. The dimension L1 may be tens of micrometers (µm), for example, 40 µm.

A dimension L2 indicates the length in the Y direction of the hexagonal second chamber 22 in a plan view viewed in the Z direction. The dimension L2 may be tens of micrometers (µm), for example, 50 µm. In the figure, the volume of the second chamber 22 is larger than the volume of the first chamber 21.

A dimension L3 indicates the width of the flow path part 20 in the Y direction. The flow path part 20 may allow the solution 30 to flow, and the dimension L3 may be a value smaller than the dimension L1 and the dimension L2, and may be, for example, 35 µm.

A dimension L4 indicates the width of the flow path part 20 in the Z direction. The flow path part 20 may allow the solution 30 to flow. The dimension L4 may be a value smaller than the dimension L1 and the dimension L2, and may be, for example, 18 µm.

A dimension L5 indicates the width of the opening part 19. The dimension L5 may be tens to hundreds of micrometers (µm), and may be, for example, 240 µm.

The solution 30 is a mixture of a target DNA fragment with a fluorescent dye (e.g., SYBR (registered trademark) Green), a primer, a DNA polymerase, and a buffer. When the solution 30 is injected into the tank part 15, the solution 30 is filled in the portion from the flow path part 20 to the second chamber 22 and the first chamber 21 via the opening part 19.

The above dimensions should be designed to meet the following. When the solution 30 is filled from the tank part 15 to the opening part 19, the flow path part 20, the second chamber 22, and the first chamber 21, the air pressure is balanced so that the solution 30 does not fall naturally from the opening of the first chamber 21 serving as a discharge nozzle in the -Z direction.

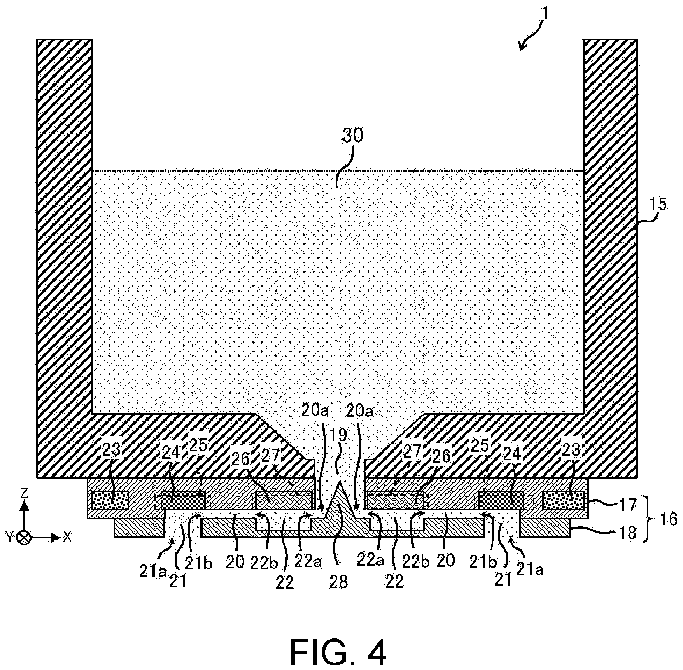

The cross section of the cartridge 1 shown in (a) of FIG. 2 may also be as shown in FIG. 4. FIG. 4 is a view showing a configuration of a cross section and a lower surface of the cartridge 1 according to a first modification example of the embodiment. Compared to FIG. 2, in FIG. 4, a partition wall 28 is added to the head film 18. Accordingly, the heat insulating property is improved between the two second chambers 22 sandwiching the partition wall 28, and adverse thermal effect can be suppressed.

Next, the circuit configuration of the cartridge 1 will be described.

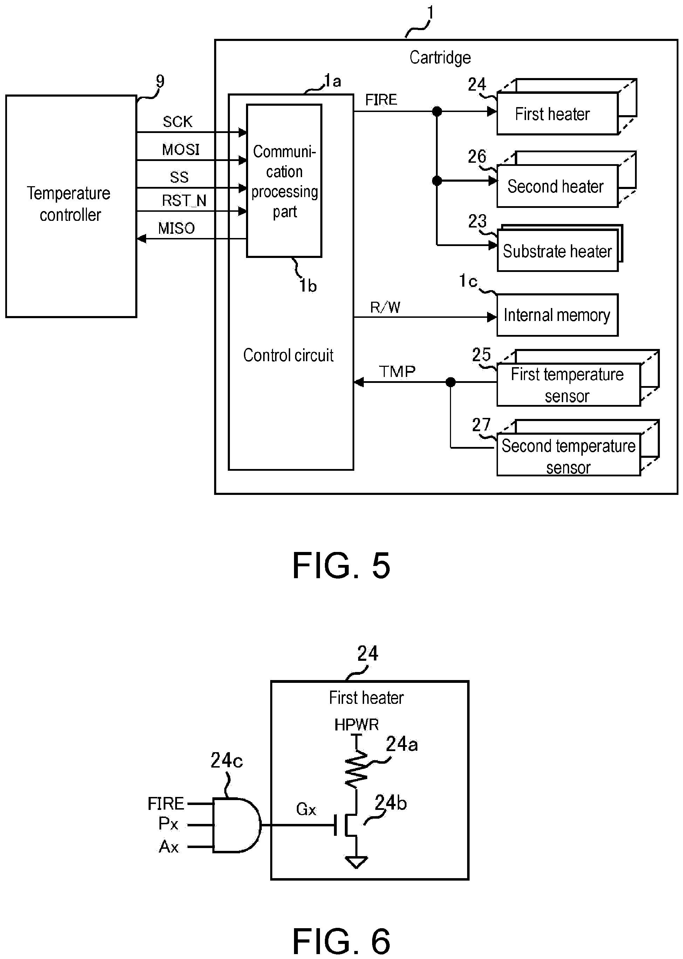

FIG. 5 is a block diagram showing a circuit configuration example of the cartridge 1 according to the embodiment. The figure also shows the temperature controller 9 and signal lines connected with the temperature controller 9. As shown in the figure, the cartridge 1 includes a control circuit 1a, a plurality of first heaters 24, a plurality of second heaters 26, a plurality of substrate heaters 23, an internal memory 1c, a plurality of first temperature sensors 25, and a plurality of second temperature sensors 27.

The control circuit 1a includes therein a communication processing part 1b.

The communication processing part 1b is connected to the temperature controller 9 by a Serial Peripheral Interface (SPI). SCK is an abbreviation for Serial Clock, which is a clock signal. MOSI is an abbreviation for Master Out Slave In, and is an address, command, data, etc. sent as serial data from the temperature controller 9 to the communication processing part 1b. SS is an abbreviation for Slave Select and is a chip select signal. RST_N is a reset signal. MISO is an abbreviation for Master In Slave Out, and is serial data from the communication processing part 1b to the temperature controller 9. The communication processing part 1b receives, from the temperature controller 9, a command for instructing heating of the first heaters 24, the second heaters 26, and the substrate heaters 23 individually or in groups, a command for instructing discharge, and the like. Further, the communication processing part 1b receives a request for temperature data for the first temperature sensors 25 and the second temperature sensors 27 individually or in groups, and transmits the requested temperature data to the temperature controller 9.

When the communication processing part 1b receives a command instructing heating, the control circuit 1a controls the corresponding first heaters 24, second heaters 26, and substrate heaters 23 to heat or discharge according to the command. Further, when the communication processing part 1b receives a command requesting temperature data, the control circuit 1a performs control so that temperature data is acquired from the corresponding first temperature sensors 25 and second temperature sensors 27, the acquired temperature data is stored in the internal memory 1c, and further, the temperature data is transmitted to the temperature controller 9 via the communication processing part 1b according to the command. FIRE in the figure is a signal instructing heat generation. R/W is a signal instructing reading/writing of the internal memory 1c. TMP is a signal indicating the temperature data.

The communication between the temperature controller 9 and the communication processing part 1b is not limited to SPI, and may be another communication method.

Next, the heating operation and the discharge operation by the first chamber 21 and the first heater 24 will be described.

FIG. 6 is a circuit diagram showing a configuration example of the first heater 24 and its periphery according to the embodiment. As shown in the figure, the first heater 24 has a heater resistor 24a and a switch transistor 24b. The heater resistor 24a generates heat when the switch transistor 24b is on. The switch transistor 24b is in an on state when a gate signal Gx is at a high level and is in an off state when the gate signal Gx is at a low level.

An address decoder 24c is a circuit in the control circuit 1a, decodes an address signal Ax and an address signal Px which indicates a group, and instructs a target first heater 24 for heating or discharging. Each of the address signal Ax and the address signal Px is actually a multi-bit signal, but are shown in the figure as 1-bit signals for the sake of simplicity.

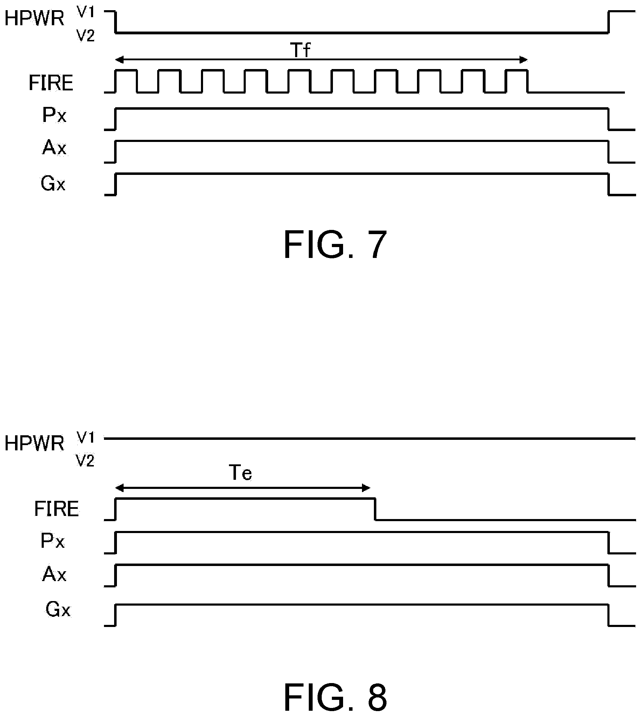

FIG. 7 is a time chart showing an operation example of heating the solution of the first chamber 21 according to the embodiment. The figure shows a heating operation example in the first heater 24 corresponding to the address signal Ax and the address signal Px. In the heating operation, a power supply voltage HPWR of the heater resistor 24a drops from a high voltage V1 to a low voltage V2, and the FIRE signal becomes a plurality of intermittent pulse signals. The heater resistor 24a generates heat intermittently according to the pulse. Accordingly, the first heater 24 heats the solution of the first chamber 21 without discharging it. In the heating operation, for example, V2 of the power supply voltage HPWR may be 4 V, and a period Tf of the intermittent pulse may be about 500 microseconds (µs). In the heating operation, the temperature controller 9 performs feedback control that repeats the heating operation and the temperature acquisition.

FIG. 8 is a time chart showing an operation example of discharging the solution of the first chamber 21 according to the embodiment. In the discharge operation of the figure, the power supply voltage HPWR of the heater resistor 24a is maintained at the high voltage V1, and the FIRE signal has one pulse having a long high level period. Accordingly, the heater resistor 24a generates more heat than in FIG. 7. As a result, the first heater 24 generates bubbles in the solution of the first chamber 21 to discharge the solution. In the discharge operation, for example, V1 of the power supply voltage HPWR may be 10 V, and a period Te of the pulse for maintaining the high level may be about 100 µs.

Next, the changes in position by the movement of the cartridge 1 in the microfluidic device 100 will be described.

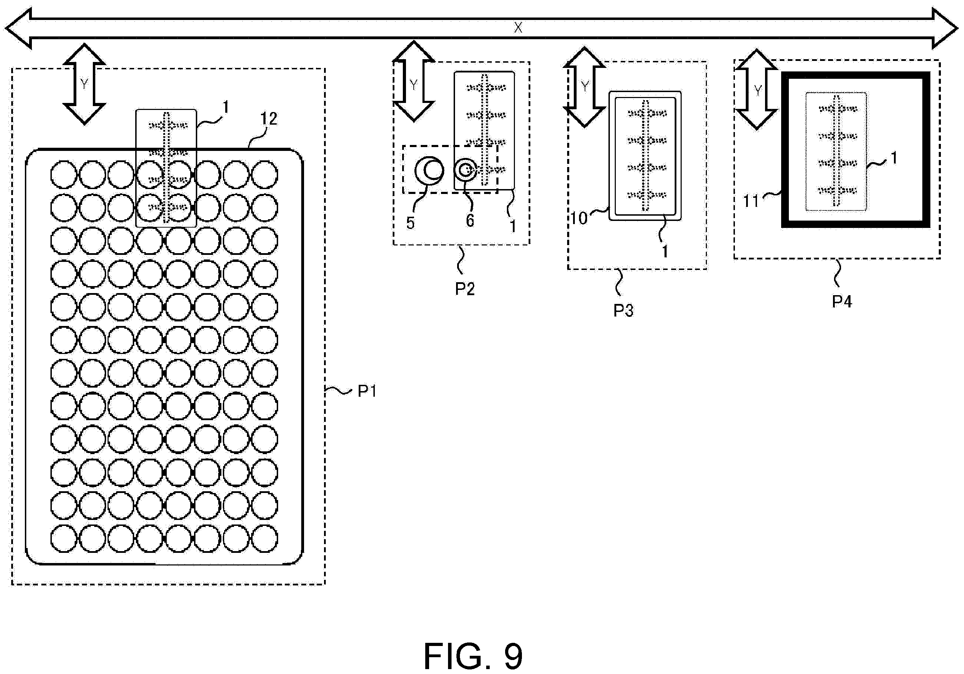

FIG. 9 is a view showing positions of moving destinations of the cartridge 1 according to the embodiment. In the figure, a position P1 indicates the position of the plate 12. A position P2 indicates the position of the optical part composed of the light source 5 and the light receiving sensor 6. A position P3 indicates the position of the cap 10. A position P4 indicates the position of the waste liquid tray 11. The position controller 4 selectively moves the cartridge 1 to these positions.

When repetitions of the thermal cycle have been completed, i.e., when the amplification of the DNA fragment is in the plateau state, the position controller 4 moves the cartridge 1 to the position P1 to dispense the solution 30 held in the first chambers 21 and the second chambers 22. Further, by finely adjusting the position at the position P1, the position controller 4 moves the discharge target, i.e., the first chamber 21 or the second chamber 22, to the position of each well in the plate 12. Accordingly, the required amount of the solution 30 can be dispensed into each well from the first chambers 21 and the second chambers 22.

To measure the fluorescence intensity for each thermal cycle, the position controller 4 moves the cartridge 1 to the position P2 to measure the fluorescence intensity of the first chamber 21. At this time, the fluorescence intensity of the second chamber 22 may also be measured. When the irradiation range of the light source 5 extends to both the first chamber 21 and the second chamber 22, the position controller 4 may have the combined fluorescence intensity of the first chamber 21 and the second chamber 22 measured.

The position controller 4 moves the cartridge 1 to the position P3 and attaches the cap 10 while the cartridge 1 is in the thermal cycle. This prevents or suppresses drying of the first chamber 21.

Further, in the measurement of the fluorescence intensity for each thermal cycle, when a defective first chamber 21 is present, the position controller 4 moves the cartridge 1 to the position P4 to replace the solution 30 of the first chamber 21 by discharging the solution 30 of the first chamber 21 to the waste liquid tray 11.

Next, the overall flow of the nucleic acid amplification operation in the microfluidic device 100 will be described.

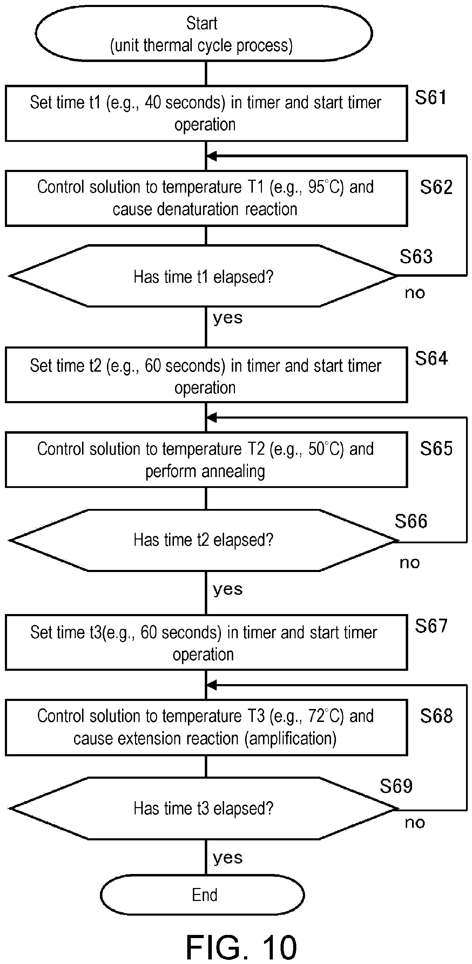

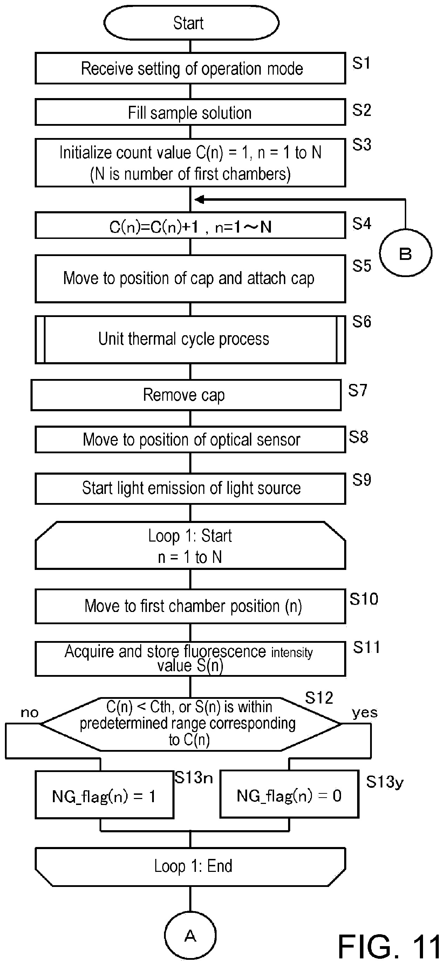

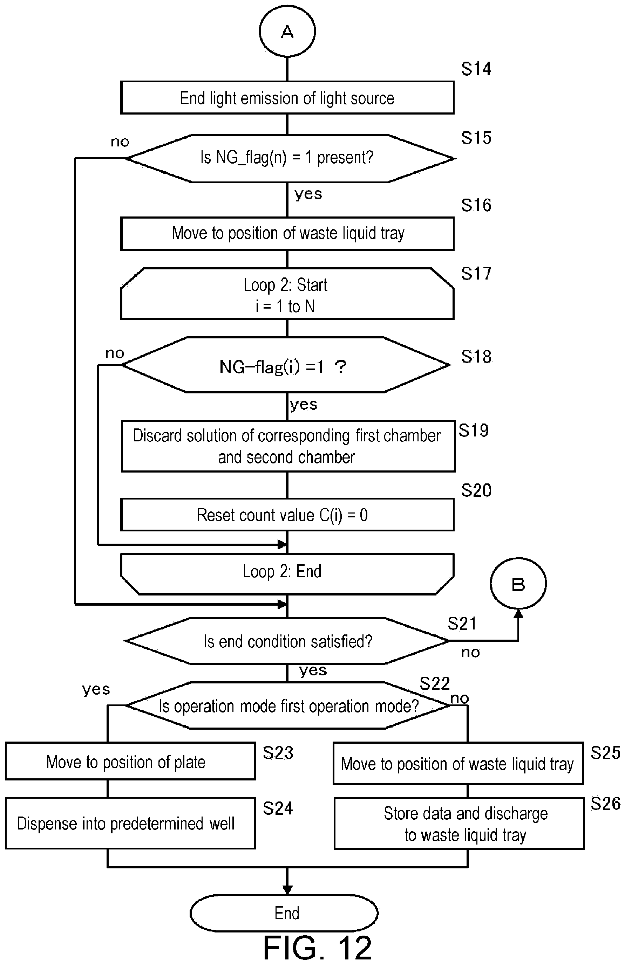

FIG. 11 is a flowchart showing an operation for nucleic acid amplification using the microfluidic device 100 according to the embodiment. FIG. 12 is a flowchart showing a continuation of the operation of FIG. 11. Further, FIG. 10 is a flowchart showing an example of a unit thermal cycle process in FIG. 11.

First, an example of a unit thermal cycle process of FIG. 10 will be described.

As shown in FIG. 10, in the unit thermal cycle process, the control part 14 first sets a time t1 in an internal timer and starts a timer operation (S61). The time t1 is, for example, 40 seconds. The control part 14 controls the held solution 30 to a temperature T1 and causes a denaturation reaction for each of the first chambers 21 and the second chambers 22 (S62). The temperature T1 is, for example, 95°C. Further, the control part 14 determines whether the time t1 has elapsed from the start of the timer operation (S63). If the time t1 has not elapsed, the process returns to step S62, and if the time t1 has elapsed, the process proceeds to step S64. Steps S61 through S63 give rise to a denaturation reaction, which is the first stage of the unit thermal cycle.

Further, the control part 14 first sets a time t2 in the internal timer and starts the timer operation (S64). The time t2 is, for example, 60 seconds. The control part 14 controls the held solution 30 to a temperature T2 and performs annealing for each of the first chambers 21 and the second chambers 22 (S65). The temperature T2 is, for example, 50°C. Further, the control part 14 determines whether the time t2 has elapsed from the start of the timer operation (S66). If the time t2 has not elapsed, the process returns to step S65, and if the time t2 has elapsed, the process proceeds to step S67. Steps S64 through S66 are annealing, which is the second stage of the unit thermal cycle.

Next, the control part 14 first sets a time t3 in the internal timer and starts the timer operation (S67). The time t3 is, for example, 60 seconds. The control part 14 controls the held solution 30 to a temperature T3 and performs an extension reaction, i.e., amplification, for each of the first chambers 21 and the second chambers 22 (S68). The temperature T3 is, for example, 72°C. Further, the control part 14 determines whether the time t3 has elapsed from the start of the timer operation (S69). If the time t3 has not elapsed, the process returns to step S68, and if the time t3 has elapsed, the unit thermal cycle process is completed. Steps S67 through S69 cause an extension reaction, which is the third stage of the unit thermal cycle.

Next, an example of the nucleic acid amplification operation will be described with reference to FIG. 11 and FIG. 12. The nucleic acid amplification operation includes so-called qPCR, and further includes a process of suppressing adverse effects when the starting material is defective.

In FIG. 11, the control part 14 first receives an operation mode of the microfluidic device 100 (S1). In this example, either a first operation mode or a second operation mode is received as the operation mode according to a user operation. The first operation mode is an operation mode in which the solution 30 containing the DNA fragment amplified by the nucleic acid amplification operation of FIG. 11 and FIG. 12 is dispensed into the plate 12. The second operation mode is an operation mode in which the solution 30 containing the DNA fragment amplified by the nucleic acid amplification operation of FIG. 11 and FIG. 12 is discarded and only the data obtained by qPCR is stored. For example, the user may select the second operation mode if qPCR may achieve the objectives of analysis and identification of the test target, and may select the first operation mode to further utilize the solution 30 amplified by qPCR.

Further, the user fills the tank part 15 with the sample solution 30 (S2). The solution 30 contains the target DNA fragment, a fluorescent dye (e.g., SYBR Green), a primer, a DNA polymerase, and a buffer. When the solution 30 is injected into the tank part 15, the solution 30 is filled in the portion from the flow path part 20 to the second chamber 22 and the first chamber 21 via the opening part 19.

Up to this point is the user preparatory stage of the nucleic acid amplification operation. After the preparatory stage, the control part 14 initializes a count value C(n) to 1 for each first chamber 21 (S3). n is the total number of first chambers 21 included in the cartridge 1. n is, for example, about 32 to 640. Further, the count value C(n) indicates a count value which is the number of repetitions of the thermal cycle of the corresponding first chamber 21.

Further, the control part 14 increments the count value C(n) by 1 for all n of n = 1 to N (S4), and moves the cartridge 1 to the position P3 of the cap 10 and attaches the cap 10 (S5). In this state, the control part 14 performs the unit thermal cycle process shown in FIG. 10 (S6).

After the unit thermal cycle process is completed, the control part 14 removes the cap 10 from the cartridge 1 (S7), moves to the position P2 of the optical part composed of the light source 5 and the light receiving sensor 6 (S8), and starts light emission of the light source 5 (S9).

In this state, the control part 14 measures and stores the fluorescence intensity for each of the N first chambers 21 and checks for defects (loop 1).

In the loop 1, the control part 14 moves the optical part to a first chamber 21 position (n) corresponding to n (S10), and measures the fluorescence intensity with the light receiving sensor 6. The control part 14 acquires a measured fluorescence intensity value S(n) and stores it in the memory 13a (S11). Further, when C(n) is smaller than a predetermined threshold value Cth, the control part 14 proceeds to S13y regardless of the fluorescence intensity value S(n), and when C(n) is equal to or greater than the predetermined threshold value Cth, the control part 14 determines whether the fluorescence intensity value S(n) is within a predetermined range defined in advance associated with the number of repetitions (S12).

Herein, the predetermined range indicates a range of the expected value for each number of repetitions calculated from the data of the known PCR amplification effect for the known DNA fragment in the solution 30. Therefore, S12 is a step of determining whether the known DNA fragment is amplified as expected from the data. Accordingly, when the fluorescence intensity value S(n) is within the predetermined range, it means that a quantitative defect or a qualitative defect is not present in the starting material of the solution 30.

In contrast, when the fluorescence intensity value S(n) is not within the predetermined range, it means that there is a high possibility that a quantitative defect or a qualitative defect of the starting material of the solution 30 has occurred. When the fluorescence intensity value S(n) is not within the predetermined range, the corresponding first chamber 21 is referred to as a defective chamber.

When C(n) is smaller than the predetermined threshold value Cth in S12, the control part 14 sets NG_flag(n) = 0 (S13y). The predetermined threshold value Cth is, for example, about 10 to 20. When the number of repetitions is equal to or less than the threshold value Cth, the fluorescence intensity is small and there is no significant difference due to the number of repetitions, so NG_flag(n) = 0 is forcibly set.

The control part 14 sets NG_flag(n) = 0 when C(n) is equal to or greater than the predetermined threshold value Cth, and the fluorescence intensity value S(n) is within a predetermined range defined in advance associated with the number of repetitions (S13y).

The control part 14 sets NG_flag(n) = 1 when C(n) is equal to or greater than the predetermined threshold value Cth, and the fluorescence intensity value S(n) is not within the predetermined range defined in advance associated with the number of repetitions (S13n).

The control part 14 executes the loop 1 process for the N first chambers 21 corresponding to n = 1 to N.

The NG_flag(n) is stored in the memory 13a in association with the count value C(n).

Next, as shown in FIG. 12, the control part 14 ends the light emission of the light source 5 after the loop 1 process is completed (S14), and determines whether NG_flag(n) = 1 stored in the memory 13a is present (S15). The control part 14 proceeds to S21 when NG_flag(n) = 1 is not present, and proceeds to S16 when NG_flag(n) = 1 is present.

Further, the control part 14 moves the cartridge 1 to the position P4 of the waste liquid tray 11 (S16). In this state, the control part 14 executes a loop 2 process (S17).

The control part 14 determines whether NG_flag(i) = 1 in the loop 2 process (S18). When the control part 14 determines that NG_flag(i) = 1 (yes in S18), the solution held in the corresponding first chamber 21 and second chamber 22 is discarded (S19). In S19, the control part 14 causes the first chamber 21 to perform a plurality of discharge operations. The total of the solution 30 in the first chamber 21, the solution 30 in the second chamber 22, and the solution 30 in the flow path part 20 is discharged to the waste liquid tray 11 by the discharge operations. Further, by this discharge, the first chamber 21, the second chamber 22, and the flow path part 20 are filled with the new solution 30 from the tank part 15.

Next, the control part 14 resets a value C(i) corresponding to the first chamber 21 to 0 (S20).

In the loop 2 process, for all defective chambers corresponding to NG_flag(n) = 1, the solution of the corresponding second chamber 22 and the flow path part 20 may also be replaced with the new solution 30 to newly start the nucleic acid amplification operation.

After the end of the loop 2 process, the control part 14 determines, for example, whether the current state satisfies an end condition (S21). Herein, the end condition may be one or more of the following: the amplification of nucleic acid is in the plateau state and is stagnant; the average of the fluorescence intensity S(n) has reached the threshold value; the number of times of executions of the unit thermal cycle process reaches a number of times obtained by adding a margin number of times to a predetermined number of times corresponding to the plateau state. The control part 14 proceeds to S4 when determining that the end condition is not satisfied, and proceeds to S22 when determining that the end condition is satisfied.

Further, the control part 14 determines whether the operation mode is the first operation mode (S22).

When it is determined to be the first operation mode, the control part 14 moves the cartridge 1 to the position P1 of the plate 12 (S23), and dispenses the solution 30 from the cartridge 1 into predetermined wells of the plate 12 (S24). The dispensed plate 12 is further subjected to other tests such as electrophoresis.

In contrast, when it is determined not to be the first operation mode, the control part 14 moves the cartridge 1 to the position P4 of the waste liquid tray 11 (S25), stores data such as the count value C(n) and the fluorescence intensity value S(n) in the memory 13a, and discharges the solution 30 to the waste liquid tray 11 (S26). In the discharge in S26, at least the solution 30 in the first chamber 21, the second chamber 22, and the flow path part 20 may be discharged.

In the nucleic acid amplification operation of FIG. 11 and FIG. 12, it is possible to reduce the adverse effect when a quantitative defect or a qualitative defect is present in the starting material contained in the solution 30 held in the first chamber 21. In other words, if a quantitative defect or a qualitative defect is present in the starting material, the control part 14 determines the defective chamber and replaces the solution 30 of the defective chamber with the new solution. That is, the first chamber 21 in which a quantitative defect or a qualitative defect is present in the starting material starts a new thermal cycle with the new solution. Therefore, among the plurality of first chambers 21, the first chambers that have adverse effect can be eliminated or reduced.

Further, since one first heater 24 corresponds to one first chamber 21, in the thermal cycle, the time required to heat the first chamber 21 to the target temperature can be shortened, and the total time required for the thermal cycle can be shortened.

Similarly, since one second heater 26 corresponds to one second chamber 22, in the thermal cycle, the time required to heat the second chamber 22 to the target temperature can be shortened, and the total time required for the thermal cycle can be shortened.

Further, since the first temperature sensor 25 is provided for each first chamber 21, the speed and accuracy of temperature control in the thermal cycle can be improved.

Similarly, since the second temperature sensor 27 is provided for each second chamber 22, the speed and accuracy of temperature control in the thermal cycle can be improved.

Further, since the substrate heater 23 is provided, for example, the semiconductor substrate 17 can be auxiliarily heated when the environment temperature is low.

Further, in the above embodiment, it has been shown as an example that, in order to set the solution 30 to the target temperature, heating is actively performed by heaters, and cooling is performed by natural cooling. However, the cooling may also be performed by active cooling instead of natural cooling. Next, a configuration example of actively cooling the solution will be described.

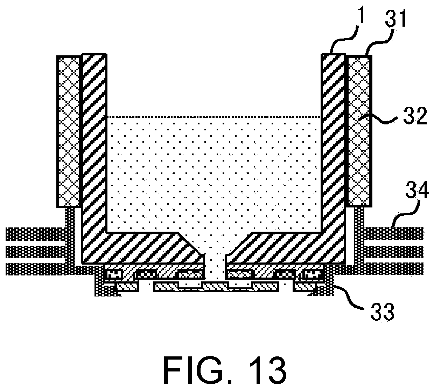

FIG. 13 is a view showing a cross-sectional configuration example of the cartridge 1 according to a second modification example of the embodiment. The figure shows a configuration example in which the cartridge 1 is held by a cartridge holder 31 in order to enhance the cooling function for the solution 30 in the cartridge 1.

The cartridge holder 31 includes a sidewall 32, a heat dissipation plate 33, and a heat dissipation fin 34.

The sidewall 32 holds the tank part 15 of the cartridge 1.

The heat dissipation plate 33 is in contact with the lower part of the tank part 15 of the cartridge 1, the semiconductor substrate 17, and the head film 18, and conducts the heat of the cartridge 1 to the heat dissipation fin 34.

The heat dissipation fin 34 dissipates heat conducted from the heat dissipation plate 33.

According to this configuration, compared to natural cooling without the heat dissipation fin 34, in a unit thermal cycle process, the time required for cooling the first chamber 21 to the target temperature can be shortened, and the total time required for the unit thermal cycle process can be further shortened.

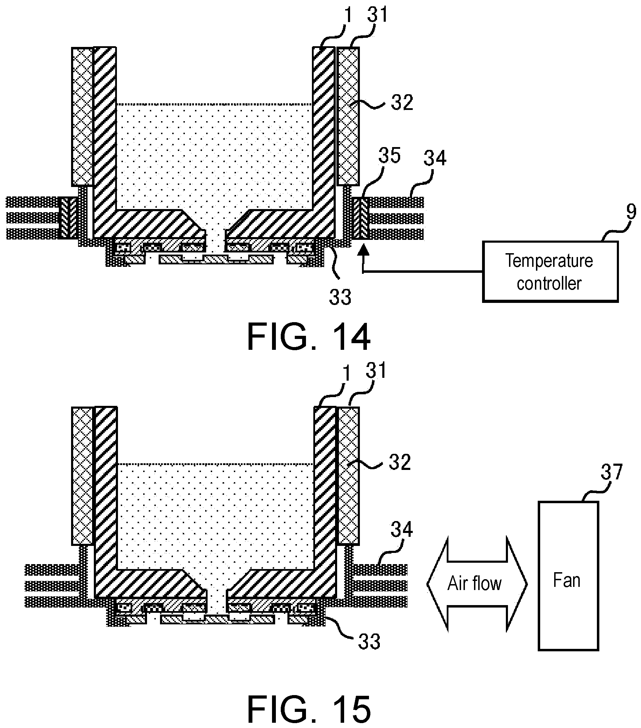

FIG. 14 is a view showing a cross-sectional configuration example of the cartridge 1 according to a third modification example of the embodiment. FIG. 14 is different from FIG. 13 in that an electronic cooling element 35 is sandwiched at an interface at which the heat dissipation plate 33 and the heat dissipation fin 34 face each other and are connected to each other.

The electronic cooling element 35 is, for example, a Peltier element, a cooling element having a semiconductor heterostructure, or the like. The Peltier element has two planar electrodes, and transfers heat to the planar electrode on the heat dissipation plate 33 side or the planar electrode on the heat dissipation fin 34 side. Further, the cooling element having a semiconductor heterostructure is an element that has a GaAs/AlₓGa₁₋ₓAs heterostructure and cools by the resonant tunneling effect and thermionic emission. The cooling capacity of the electronic cooling element 35 may be controlled by the control of the temperature controller 9.

Accordingly, the configuration of FIG. 14 has a higher cooling capacity than that of FIG. 13. The total time required for the unit thermal cycle process can be further shortened. The electronic cooling element 35 may also be a cooling element different from the Peltier element and the cooling element having a semiconductor heterostructure.

FIG. 15 is a view showing a cross-sectional configuration example of the cartridge 1 according to a fourth modification example of the embodiment. FIG. 15 is different from FIG. 13 in that it includes a fan 37. The fan 37 blows air to the heat dissipation fin 34 or takes in air from the heat dissipation fin 34.

Accordingly, the configuration of FIG. 15 has a higher cooling capacity than that of FIG. 13. The total time required for the unit thermal cycle process can be further shortened.

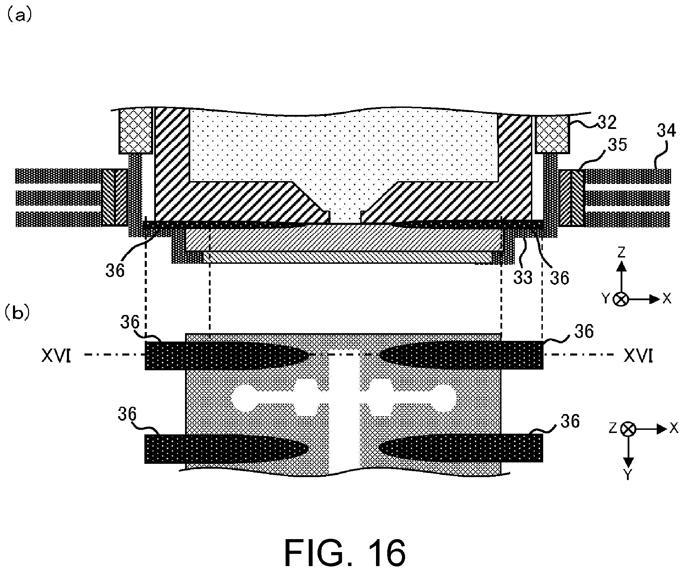

FIG. 16 is a view showing a configuration example of a cross section and a lower surface of the cartridge 1 according to a fifth modification example of the embodiment. FIG. 16 is different from FIG. 14 in that a heat conductive member 36 is added.

The heat conductive member 36 is a needle-shaped member that is inserted at an interface between the tank part 15 and the thermal head part 16 and conducts heat to the heat dissipation fin 34, and is in contact with the heat dissipation plate 33.

Accordingly, the configuration of FIG. 16 has a higher cooling capacity than that of FIG. 14. The total time required for the unit thermal cycle process can be further shortened.

Although FIG. 13 to FIG. 16 have shown configuration examples in which the cartridge 1 and the cartridge holder 31 are separate bodies, the cartridge holder 31 may also be integrally formed with the cartridge 1. Further, the heat dissipation fin 34 may also be integrally formed with the cartridge 1. Similarly, the electronic cooling element 35 may also be integrally formed with the cartridge 1.

In each of the above embodiments, each component may be configured with dedicated hardware or may be realized by executing a software program suitable for each component. Each component may be realized by a program execution part such as a CPU or a processor which reads and executes a software program recorded on a recording medium such as a hard disk or a semiconductor memory.

Although the microfluidic device 100 and the nucleic acid amplification method according to the embodiment of the disclosure have been described above, the disclosure is not limited to the above embodiment.

Further, specifically, each of the above devices may be configured as a computer system composed of a microprocessor, a ROM, a RAM, a hard disk drive, a display unit, a keyboard, a mouse, and the like. Computer programs are stored in the RAM or the hard disk drive. Each device achieves its function by operation of the microprocessor according to the computer program. Herein, the computer program is configured by combining a plurality of instruction codes indicating commands to a computer in order to achieve a predetermined function.

Further, some or all of the components that form each of the above devices may be composed of one system LSI (large-scale integration). A system LSI is a super-multifunctional LSI manufactured by integrating a plurality of components on one chip, and, for example, includes a computer system configured to include a microprocessor, a ROM, a RAM, and the like. In this case, the computer program is stored in the ROM. The system LSI achieves its function by operation of the microprocessor according to the computer program.

Furthermore, some or all of the components forming each of the above devices may be composed of an IC card or a separate module that may be attached to and detached from each device. The IC card or the module is a computer system composed of a microprocessor, a ROM, a RAM, and the like. The IC card or the module may include the above super-multifunctional LSI. The IC card or the module achieves its function by operation of the microprocessor according to the computer program. The IC card or the module may have tamper resistance.

Further, the disclosure may be the methods shown above. Further, the disclosure may be computer programs that realize these methods by a computer, or may be digital signals composed of the above computer programs.

Further, the disclosure may record the above computer program or the above digital signal to a non-transitory computer-readable recording medium, such as a flexible disk, a hard disk, a CD-ROM, an MO, a DVD, a DVD-ROM, a DVD-RAM, a BD (Blu-ray (registered trademark)) disc, a semiconductor memory, or the like. Further, it may be the digital signal recorded on these non-transitory recording media.

Further, the disclosure may transmit the computer program or the digital signal via a telecommunication line, a wireless or wired communication line, a network represented by the Internet, data broadcasting, or the like.

Further, the disclosure may be a computer system including a microprocessor and a memory, the memory may store the above computer program, and the microprocessor may operate according to the computer program.

Further, by recording the programs or the digital signals on the non-transitory recording medium and transferring the same, or by transferring the programs or the digital signals via the network or the like, they may be executed by another independent computer system.

Further, the above embodiment and the above modification examples may be combined with each other.

### [Industrial applicability]

The disclosure may be used in devices that amplify nucleic acids.

### Description of Reference Numerals

- 1: cartridge
- 1a: control circuit
- 1b: communication processing part
- 2, 3: motor
- 4: position controller
- 5: light source
- 6: light receiving sensor
- 7: light source controller
- 8: fluorescence processing part
- 9: temperature controller
- 10: cap
- 11: waste liquid tray
- 12: plate
- 13: system controller
- 14: control part
- 15: tank part
- 16: thermal head part
- 17: semiconductor substrate
- 18: head film
- 19: opening part
- 20: flow path part
- 20a: third opening
- 21: first chamber
- 21a: first opening
- 21b: second opening
- 22a: fourth opening
- 22b: fifth opening
- 22: second chamber
- 23: substrate heater
- 24: first heater
- 24a: heater resistor
- 24b: switch transistor
- 24c: address decoder
- 25: first temperature sensor
- 26: second heater
- 27: second temperature sensor
- 28: partition wall
- 30: solution
- 31: cartridge holder
- 32: sidewall
- 33: heat dissipation plate
- 34: heat dissipation fin
- 35: electronic cooling element
- 37: fan
- 36: heat conductive member
- 100: microfluidic device
- P1, P2, P3, P4: position

## Claims

1. A microfluidic device (100) for amplifying a nucleic acid, comprising:
a cartridge (1) configured to store a solution (30) containing the nucleic acid;
a light source (5);
a light receiving sensor (6); and
a control part (14) controlling the cartridge (1), the light source (5) and the light receiving sensor (6),
wherein the cartridge (1) comprises:
a tank part (15) storing the solution;
a plurality of first chambers (21) holding the solution from the tank part (15);
a plurality of first heaters (24) corresponding to the first chambers (21); and
a plurality of flow path parts (20) connecting the tank part (15) and the first chambers (21), wherein each of the first chambers (21) comprises:
a first opening (21a) connected to the flow path part (20); and
a second opening (21b) for discharging the solution held in the first chamber (21),
the light source (5) is configured to irradiate light to the first chambers (21),
the light receiving sensor (6) is configured to measure a fluorescence intensity of each of the first chambers (21), and
the control part (14) is configured to:
control execution of a thermal cycle which changes a temperature of the first chambers (21),
count a number of repetitions of the thermal cycle for each of the first chambers (21) and store a count value,
acquire from the light receiving sensor (6) the fluorescence intensity of each of the first chambers (21) for each thermal cycle,
determine whether the acquired fluorescence intensity of each of the first chambers (21) is within a predetermined range defined in advance for each number of repetitions, and
determine the first chamber (21) to be a defective chamber when it is determined that the fluorescence intensity is not within the predetermined range, and
the microfluid device (100) being **characterized in that**:
the control part (14) is further configured to reset the count value of the defective chamber, discharge the solution from the defective chamber, and fill the defective chamber with a new solution from the tank part (15).

2. The microfluidic device (100) according to claim 1, wherein
the control part (14) is configured to cause the first heater (24) corresponding to the defective chamber to generate a bubble in the solution in the defective chamber so as to discharge the solution of the defective chamber from the second opening (21b) by generation of the bubble, and fills the new solution to the defective chamber from the tank part (15) via the flow path part (20) and the first opening (21a).

3. The microfluidic device (100) according to claim 1 or 2, wherein the cartridge (1) comprises a plurality of first temperature sensors (25) corresponding to the first chambers (21), and
the control part (14) is configured to acquire data indicating a temperature from the first temperature sensors (25) in the thermal cycle and manages a temperature of each of the first chambers (21).

4. The microfluidic device (100) according to claim 2, wherein the cartridge (1) comprises a thermal head part (16),
the thermal head part (16) comprises a semiconductor substrate (17) and a head film (18) bonded to the semiconductor substrate (17),
the semiconductor substrate (17) comprises the first heater (24) and the flow path part (20), and
the first chamber (21) and the flow path part (20) are formed as a gap formed by bonding the semiconductor substrate (17) and the head film (18) together.

5. The microfluidic device (100) according to claim 4, wherein the semiconductor substrate (17) comprises a substrate heater (23).

6. The microfluidic device (100) according to claim 2, wherein the cartridge (1) comprises:
a plurality of second chambers (22); and
a plurality of second heaters (26) corresponding to the second chambers (22), and
each of the second chambers (22) is located in the middle of the flow path part (20) and is provided at a position between the first opening (21a) of the first chamber (21) and a connection portion between the tank part (15) and the flow path part (20).

7. The microfluidic device (100) according to claim 6, wherein the control part (14) is configured to discharge, via the defective chamber, the solution of the second chamber (22) that is connected to the defective chamber via the flow path part (20), and fills the second chamber (22) with the new solution from the tank part (15).

8. The microfluidic device (100) according to claim 6 or 7, wherein a volume of the second chamber (22) is larger than a volume of the first chamber (21).

9. The microfluidic device (100) according to any one of claims 6 to 8, wherein the cartridge (1) comprises a plurality of second temperature sensors (27) corresponding to the second chambers (22), and
the control part (14) is configured to acquire data indicating a temperature from the second temperature sensors (27) in the thermal cycle and manages a temperature of each of the second chambers (22).

10. The microfluidic device (100) according to claim 4, wherein the cartridge (1) comprises a heat dissipation fin (34) for cooling the thermal head part (16).

11. The microfluidic device (100) according to claim 10, wherein the cartridge (1) comprises a fan (37) for cooling the heat dissipation fin (34).

12. The microfluidic device (100) according to claim 10 or 11, wherein the cartridge (1) comprises an electronic cooling element (35) for cooling the heat dissipation fin (34).

13. The microfluidic device (100) according to any one of claims 10 to 12, wherein the cartridge (1) comprises a needle-shaped heat conductive member (36) that is inserted at an interface between the tank part (15) and the thermal head part (16) to conduct heat to the heat dissipation fin (34).

14. The microfluidic device (100) according to any one of claims 1 to 13, further comprising a cap (10) covering the first chambers (21).

15. The microfluidic device (100) according to any one of claims 1 to 14, further comprising a waste liquid tray (11) receiving the solution discharged from the defective chamber.

16. The microfluidic device (100) according to any one of claims 1 to 15, further comprising a plate (12) having a plurality of wells that receive the solution discharged from the first chambers (21) excluding the defective chamber, after completion of a predetermined number of thermal cycles.

17. The microfluidic device (100) according to any one of claims 1 to 16, further comprising a movement control part configured to move the cartridge (1) at least one-dimensionally.

18. A nucleic acid amplification method in a microfluidic device (100), the microfluidic device (100) comprising:
a cartridge (1) configured to store a solution (30) containing a nucleic acid;
a light source (5);
a light receiving sensor (6); and
a control part (14) controlling the cartridge (1), the light source (5) and the light receiving sensor (6),
wherein the cartridge (1) comprises:
a tank part (15) storing the solution;
a plurality of first chambers (21) holding the solution from the tank part (15);
a plurality of first heaters (24) corresponding to the first chambers (21); and
a plurality of flow path parts (20) connecting the tank part (15) and the first chambers (21), wherein each of the first chambers (21) comprises:
a first opening (21a) connected to the flow path part (20); and
a second opening (21b) for discharging the solution held in the first chamber (21),
the light source (5) is configured to irradiate light to the first chambers (21),
the light receiving sensor (6) is configured to measure a fluorescence intensity of each of the first chambers (21), and
the nucleic acid amplification method comprises:
controlling a thermal cycle which changes a temperature of the first chambers (21);
counting a number of repetitions of the thermal cycle for each of the first chambers (21) and storing a count value;
acquiring from the light receiving sensor (6) the fluorescence intensity of each of the first chambers (21) for each thermal cycle;
determining whether the acquired fluorescence intensity of each of the first chambers (21) is within a predetermined range defined in advance for each number of repetitions; and
determining the first chamber (21) to be a defective chamber when it is determined that the fluorescence intensity is not within the predetermined range;
the nucleic acid amplification method is **characterized by**:
resetting the count value of the defective chamber, discharging the solution from the defective chamber, and filling the defective chamber with a new solution from the tank part (15).

## Patentansprüche

1. Mikrofluidische Vorrichtung (100) zur Amplifikation einer Nukleinsäure, umfassend:
eine Patrone (1), die so konfiguriert ist, dass sie eine Lösung (30), die die Nukleinsäure enthält, aufbewahrt;
eine Lichtquelle (5);
einen Lichtempfangssensor (6); und
ein Steuerteil (14), das die Patrone (1), die Lichtquelle (5) und den Lichtempfangssensor (6) steuert,
wobei die Patrone (1) umfasst:
ein Tankteil (15), in dem die Lösung aufbewahrt wird;
eine Vielzahl von ersten Kammern (21), die die Lösung aus dem Tankteil (15) halten;
eine Vielzahl von ersten Heizelementen (24), die den ersten Kammern (21) entsprechen; und
eine Vielzahl von Strömungswegteilen (20), die das Tankteil (15) und die ersten Kammern (21) verbinden, wobei jede der ersten Kammern (21) umfasst:
eine erste Öffnung (21a), die mit dem Strömungswegteil (20) verbunden ist; und
eine zweite Öffnung (21b) zum Entleeren der in der ersten Kammer (21) enthaltenen Lösung,
die Lichtquelle (5) so konfiguriert ist, dass sie die ersten Kammern (21) mit Licht bestrahlt,
der Lichtempfangssensor (6) so konfiguriert ist, dass er eine Fluoreszenzintensität jeder der ersten Kammern (21) misst, und
das Steuerteil (14) konfiguriert ist zum:
Steuern der Ausführung eines thermischen Zyklus, der die Temperatur der ersten Kammern (21) verändert,
Zählen einer Anzahl von Wiederholungen des thermischen Zyklus für jede der ersten Kammern (21) und Speichern eines Zählwerts,
Erfassen, von dem Lichtempfangssensor (6), der Fluoreszenzintensität jeder der ersten Kammern (21) für jeden thermischen Zyklus,
Bestimmen, ob die erfasste Fluoreszenzintensität jeder der ersten Kammern (21) innerhalb eines vorgegebenen Bereichs liegt, der im Voraus für jede Anzahl von Wiederholungen definiert wurde, und
Bestimmen der ersten Kammer (21) als eine defekte Kammer, wenn bestimmt wird, dass die Fluoreszenzintensität nicht innerhalb des vorgegebenen Bereichs liegt, und
die mikrofluidische Vorrichtung (100) **dadurch gekennzeichnet ist, dass**:
das Steuerteil (14) ferner so konfiguriert ist, dass es den Zählwert der defekten Kammer zurücksetzt, die Lösung aus der defekten Kammer ablässt und die defekte Kammer mit einer neuen Lösung aus dem Tankteil (15) füllt.

2. Mikrofluidische Vorrichtung (100) gemäß Anspruch 1, wobei
das Steuerteil (14) so konfiguriert ist, dass es das erste Heizelementen (24), das der defekten Kammer entspricht, veranlasst, eine Blase in der Lösung in der defekten Kammer zu erzeugen, um die Lösung der defekten Kammer aus der zweiten Öffnung (21b) durch Erzeugung der Blase abzulassen, und die neue Lösung in die defekte Kammer aus dem Tankteil (15) über das Strömungswegteil (20) und die erste Öffnung (21a) einfüllt.

3. Mikrofluidische Vorrichtung (100) gemäß Anspruch 1 oder 2, wobei die Patrone (1) eine Vielzahl von ersten Temperatursensoren (25) umfasst, die den ersten Kammern (21) entsprechen, und
das Steuerteil (14) so konfiguriert ist, dass es Daten erfasst, die eine Temperatur von den ersten Temperatursensoren (25) im thermischen Zyklus anzeigen, und eine Temperatur jeder der ersten Kammern (21) verwaltet.

4. Mikrofluidische Vorrichtung (100) gemäß Anspruch 2, wobei die Patrone (1) ein Thermokopfteil (16) umfasst,
das Thermokopfteil (16) ein Halbleitersubstrat (17) und einen auf das Halbleitersubstrat (17) verklebten Kopffilm (18) umfasst,
das Halbleitersubstrat (17) das erste Heizelementen (24) und das Strömungswegteil (20) umfasst, und
die erste Kammer (21) und das Strömungswegteil (20) als ein Spalt ausgebildet sind, der durch Zusammenkleben des Halbleitersubstrats (17) und des Kopffilms (18) gebildet wird.

5. Mikrofluidische Vorrichtung (100) gemäß Anspruch 4, wobei das Halbleitersubstrat (17) eine Substratheizelement (23) umfasst.

6. Mikrofluidische Vorrichtung (100) gemäß Anspruch 2, wobei die Patrone (1) umfasst:
eine Vielzahl von zweiten Kammern (22); und
eine Vielzahl von zweiten Heizelementen (26), die den zweiten Kammern (22) entsprechen, und
jede der zweiten Kammern (22) sich in der Mitte des Strömungswegteils (20) befindet und an einer Position zwischen der ersten Öffnung (21a) der ersten Kammer (21) und einem Verbindungsabschnitt zwischen dem Tankteil (15) und dem Strömungswegteil (20) vorgesehen ist.

7. Mikrofluidische Vorrichtung (100) gemäß Anspruch 6, wobei das Steuerteil (14) so konfiguriert ist, dass es die Lösung der zweiten Kammer (22), die über das Strömungswegteil (20) mit der defekten Kammer verbunden ist, über die defekte Kammer abgibt und die zweite Kammer (22) mit der neuen Lösung aus dem Tankteil (15) füllt.

8. Mikrofluidische Vorrichtung (100) gemäß Anspruch 6 oder 7, wobei ein Volumen der zweiten Kammer (22) größer ist als ein Volumen der ersten Kammer (21).

9. Mikrofluidische Vorrichtung (100) gemäß irgendeinem der Ansprüche 6 bis 8, wobei die Patrone (1) eine Vielzahl von zweiten Temperatursensoren (27) umfasst, die den zweiten Kammern (22) entsprechen, und
das Steuerteil (14) so konfiguriert ist, dass es Daten erfasst, die eine Temperatur von den zweiten Temperatursensoren (27) im thermischen Zyklus anzeigen, und eine Temperatur jeder der zweiten Kammern (22) verwaltet.

10. Mikrofluidische Vorrichtung (100) gemäß Anspruch 4, wobei die Patrone (1) eine Wärmeableitungsrippe (34) zur Kühlung des Thermokopfteils (16) umfasst.

11. Mikrofluidische Vorrichtung (100) gemäß Anspruch 10, wobei die Patrone (1) ein Gebläse (37) zur Kühlung der Wärmeableitungsrippe (34) umfasst.

12. Mikrofluidische Vorrichtung (100) gemäß Anspruch 10 oder 11, wobei die Patrone (1) ein elektronisches Kühlelement (35) zur Kühlung der Wärmeableitungsrippe (34) umfasst.

13. Mikrofluidische Vorrichtung (100) gemäß irgendeinem der Ansprüche 10 bis 12, wobei die Patrone (1) ein nadelförmiges wärmeleitendes Element (36) umfasst, das an einer Schnittstelle zwischen dem Tankteil (15) und dem Thermokopfteil (16) eingesetzt ist, um Wärme zu der Wärmeableitungsrippe (34) zu leiten.

14. Mikrofluidische Vorrichtung (100) gemäß irgendeinem der Ansprüche 1 bis 13, die ferner eine Kappe (10) umfasst, die die ersten Kammern (21) abdeckt.

15. Mikrofluidische Vorrichtung (100) gemäß irgendeinem der Ansprüche 1 bis 14, ferner umfassend eine Abfallflüssigkeitsschale (11), die die aus der defekten Kammer abgelassene Lösung aufnimmt.

16. Mikrofluidische Vorrichtung (100) gemäß irgendeinem der Ansprüche 1 bis 15, ferner umfassend eine Platte (12) mit einer Vielzahl von Vertiefungen, die die aus den ersten Kammern (21) mit Ausnahme der defekten Kammer abgegebene Lösung nach Abschluss einer vorbestimmten Anzahl von thermischen Zyklen aufnehmen.

17. Mikrofluidische Vorrichtung (100) gemäß irgendeinem der Ansprüche 1 bis 16, ferner umfassend ein Bewegungssteuerungselement, das so konfiguriert ist, dass es die Patrone (1) mindestens eindimensional bewegt.

18. Nukleinsäure-Amplifikationsverfahren in einer mikrofluidischen Vorrichtung (100), wobei die mikrofluidische Vorrichtung (100) umfasst:
eine Patrone (1), die so konfiguriert ist, dass sie eine Lösung (30), die die Nukleinsäure enthält, aufbewahrt;
eine Lichtquelle (5);
einen Lichtempfangssensor (6); und
ein Steuerteil (14), das die Patrone (1), die Lichtquelle (5) und den Lichtempfangssensor (6) steuert,
wobei die Patrone (1) umfasst:
ein Tankteil (15), in dem die Lösung aufbewahrt wird;
eine Vielzahl von ersten Kammern (21), die die Lösung aus dem Tankteil (15) halten;
eine Vielzahl von ersten Heizelementen (24), die den ersten Kammern (21) entsprechen; und
eine Vielzahl von Strömungswegteilen (20), die das Tankteil (15) und die ersten Kammern (21) verbinden, wobei jede der ersten Kammern (21) umfasst:
eine erste Öffnung (21a), die mit dem Strömungswegteil (20) verbunden ist; und
eine zweite Öffnung (21b) zum Entleeren der in der ersten Kammer (21) enthaltenen Lösung,
die Lichtquelle (5) so konfiguriert ist, dass sie die ersten Kammern (21) mit Licht bestrahlt,
der Lichtempfangssensor (6) so konfiguriert ist, dass er eine Fluoreszenzintensität jeder der ersten Kammern (21) misst, und
das Nukleinsäure-Amplifikationsverfahren umfasst:
Steuern eines thermischen Zyklus, der die Temperatur der ersten Kammern (21) verändert;
Zählen einer Anzahl von Wiederholungen des thermischen Zyklus für jede der ersten Kammern (21) und Speichern eines Zählwerts;
Erfassen, von dem Lichtempfangssensor (6), der Fluoreszenzintensität jeder der ersten Kammern (21) für jeden thermischen Zyklus;
Bestimmen, ob die erfasste Fluoreszenzintensität jeder der ersten Kammern (21) innerhalb eines vorbestimmten Bereichs liegt, der im Voraus für jede Anzahl von Wiederholungen definiert wurde; und
Bestimmen, dass die erste Kammer (21) eine defekte Kammer ist, wenn bestimmt wird, dass die Fluoreszenzintensität nicht innerhalb des vorgegebenen Bereichs liegt;
Wobei das Nukleinsäure-Amplifikationsverfahren **gekennzeichnet ist durch**:
Rücksetzen des Zählwerts der defekten Kammer, Entleeren der Lösung aus der defekten Kammer und Befüllen der defekten Kammer mit einer neuen Lösung aus dem Tankteil (15).

## Revendications

1. Un dispositif microfluidique (100) pour amplifier un acide nucléique, comprenant :
une cartouche (1) configurée pour stocker une solution (30) contenant l'acide nucléique ;
une source de lumière (5) ;
un capteur (6) de réception de lumière ; et
une partie de commande (14) commandant la cartouche (1), la source de lumière (5) et le capteur (6) de réception de lumière,
la cartouche (1) comprenant :
une partie réservoir (15) stockant la solution ;
une pluralité de premières chambres (21) contenant la solution provenant de la partie réservoir (15) ;
une pluralité de premiers éléments chauffants (24) correspondant aux premières chambres (21) ; et
une pluralité de parties (20) de trajet d'écoulement, reliant la partie réservoir (15) et les premières chambres (21), chacune des premières chambres (21) comprenant :
une première ouverture (21a) reliée à la partie (20) de trajet d'écoulement ; et
une deuxième ouverture (21b) pour évacuer la solution retenue dans la première chambre (21),
la source de lumière (5) est configurée pour émettre de la lumière vers les premières chambres (21),
le capteur (6) de réception de lumière est configuré pour mesurer une intensité de fluorescence de chacune des premières chambres (21), et
la partie de commande (14) est configurée pour :
commander l'exécution d'un cycle thermique qui modifie la température des premières chambres (21),
compter un nombre de répétitions du cycle thermique pour chacune des premières chambres (21) et stocker une valeur de comptage,
acquérir auprès du capteur (6) de réception de lumière l'intensité de fluorescence de chacune des premières chambres (21) pour chaque cycle thermique,
déterminer si l'intensité de fluorescence acquise de chacune des premières chambres (21) se situe dans une gamme prédéterminée définie à l'avance pour chaque nombre de répétitions, et
déterminer la première chambre (21) comme étant une chambre défectueuse lorsqu'il est déterminé que l'intensité de fluorescence n'est pas dans la gamme prédéterminée, et
le dispositif microfluidique (100) étant **caractérisé en ce que** :
la partie de commande (14) est en outre configurée pour réinitialiser la valeur de comptage de la chambre défectueuse, évacuer la solution de la chambre défectueuse et remplir la chambre défectueuse avec une nouvelle solution provenant de la partie réservoir (15).

2. Le dispositif microfluidique (100) selon la revendication 1, dans lequel
la partie de commande (14) est configurée pour amener le premier élément chauffant (24) correspondant à la chambre défectueuse à générer une bulle dans la solution présente dans la chambre défectueuse de manière à évacuer la solution de la chambre défectueuse depuis la deuxième ouverture (21b) par génération de la bulle, et remplit la nouvelle solution vers la chambre défectueuse à partir de la partie réservoir (15) via la partie (20) de trajet d'écoulement et la première ouverture (21a).

3. Le dispositif microfluidique (100) selon la revendication 1 ou la revendication 2, dans lequel la cartouche (1) comprend une pluralité de premiers capteurs de température (25) correspondant aux premières chambres (21), et
la partie de commande (14) est configurée pour acquérir des données indiquant une température provenant des premiers capteurs de température (25) dans le cycle thermique et gère une température de chacune des premières chambres (21).

4. Le dispositif microfluidique (100) selon la revendication 2, dans lequel la cartouche (1) comprend une partie tête thermique (16),
la partie tête thermique (16) comprend un substrat semi-conducteur (17) et un film de tête (18) lié au substrat semi-conducteur (17),
le substrat semi-conducteur (17) comprend le premier élément chauffant (24) et la partie (20) de trajet d'écoulement, et
la première chambre (21) et la partie (20) de trajet d'écoulement sont formées sous la forme d'un espace formé en liant le substrat semi-conducteur (17) et le film de tête (18) l'un à l'autre.

5. Le dispositif microfluidique (100) selon la revendication 4, dans lequel le substrat semi-conducteur (17) comprend un élément chauffant (23) de substrat.

6. Le dispositif microfluidique (100) selon la revendication 2, dans lequel la cartouche (1) comprend :
une pluralité de deuxièmes chambres (22) ; et
une pluralité de deuxièmes éléments chauffants (26) correspondant aux deuxièmes chambres (22), et
chacune des deuxièmes chambres (22) est située au milieu de la partie (20) de trajet d'écoulement et est disposée à une position entre la première ouverture (21a) de la première chambre (21) et une partie de connexion entre la partie réservoir (15) et la partie (20) de trajet d'écoulement.

7. Le dispositif microfluidique (100) selon la revendication 6, dans lequel la partie de commande (14) est configurée pour évacuer, via la chambre défectueuse, la solution de la deuxième chambre (22) qui est connectée à la chambre défectueuse via la partie (20) de chemin d'écoulement, et remplit la deuxième chambre (22) avec la nouvelle solution provenant de la partie réservoir (15).

8. Le dispositif microfluidique (100) selon la revendication 6 ou la revendication 7, dans lequel un volume de la deuxième chambre (22) est plus grand qu'un volume de la première chambre (21).

9. Le dispositif microfluidique (100) selon l'une quelconque des revendications 6 à 8, dans lequel la cartouche (1) comprend une pluralité de deuxièmes capteurs de température (27) correspondant aux deuxièmes chambres (22), et
la partie de commande (14) est configurée pour acquérir des données indiquant une température provenant des deuxièmes capteurs de température (27) dans le cycle thermique et gère une température de chacune des deuxièmes chambres (22).

10. Le dispositif microfluidique (100) selon la revendication 4, dans lequel la cartouche (1) comprend une ailette de dissipation thermique (34) pour refroidir la partie tête thermique (16).

11. Le dispositif microfluidique (100) selon la revendication 10, dans lequel la cartouche (1) comprend un ventilateur (37) pour refroidir l'ailette de dissipation thermique (34).

12. Le dispositif microfluidique (100) selon la revendication 10 ou la revendication 11, dans lequel la cartouche (1) comprend un élément de refroidissement électronique (35) pour refroidir l'ailette de dissipation thermique (34).

13. Le dispositif microfluidique (100) selon l'une quelconque des revendications 10 à 12, dans lequel la cartouche (1) comprend un élément conducteur de chaleur en forme d'aiguille (36) qui est inséré au niveau d'une interface entre la partie réservoir (15) et la partie tête thermique (16) pour conduire la chaleur vers l'ailette de dissipation de chaleur (34).

14. Le dispositif microfluidique (100) selon l'une quelconque des revendications 1 à 13, comprenant en outre un couvercle (10) recouvrant les premières chambres (21).

15. Le dispositif microfluidique (100) selon l'une quelconque des revendications 1 à 14, comprenant en outre un bac (11) à liquides résiduaires recevant la solution évacuée de la chambre défectueuse.

16. Le dispositif microfluidique (100) selon l'une quelconque des revendications 1 à 15, comprenant en outre une plaque (12) comportant une pluralité de puits qui reçoivent la solution évacuée des premières chambres (21) à l'exclusion de la chambre défectueuse, après achèvement d'un nombre prédéterminé de cycles thermiques.

17. Le dispositif microfluidique (100) selon l'une quelconque des revendications 1 à 16, comprenant en outre une partie de commande de mouvement configurée pour déplacer la cartouche (1) au moins de manière unidimensionnelle.

18. Un procédé d'amplification d'acide nucléique dans un dispositif microfluidique (100), le dispositif microfluidique (100) comprenant :
une cartouche (1) configurée pour stocker une solution (30) contenant un acide nucléique ;
une source de lumière (5) ;
un capteur (6) de réception de lumière ; et
une partie de commande (14) commandant la cartouche (1), la source de lumière (5) et le capteur (6) de réception de lumière,
la cartouche (1) comprenant :
une partie réservoir (15) stockant la solution ;
une pluralité de premières chambres (21) contenant la solution provenant de la partie réservoir (15) ;
une pluralité de premiers éléments chauffants (24) correspondant aux premières chambres (21) ; et
une pluralité de parties (20) de trajet d'écoulement reliant la partie réservoir (15) et les premières chambres (21), chacune des premières chambres (21) comprenant :
une première ouverture (21a) reliée à la partie (20) de trajet d'écoulement ; et
une deuxième ouverture (21b) pour évacuer la solution contenue dans la première chambre (21),
la source de lumière (5) est configurée pour émettre de la lumière vers les premières chambres (21),
le capteur (6) de réception de lumière est configuré pour mesurer une l'intensité de fluorescence de chacune des premières chambres (21), et
le procédé d'amplification d'acide nucléique comprend :
le fait de commander un cycle thermique qui modifie la température des premières chambres (21) ;
le fait de compter un nombre de répétitions du cycle thermique pour chacune des premières chambres (21) et stocker une valeur de comptage ;
le fait d'acquérir à partir du capteur (6) de réception de lumière l'intensité de fluorescence de chacune des premières chambres (21) pour chaque cycle thermique ;
le fait de déterminer si l'intensité de fluorescence acquise de chacune des premières chambres (21) se situe dans une gamme prédéterminée définie à l'avance pour chaque nombre de répétitions ; et
le fait de déterminer la première chambre (21) comme étant une chambre défectueuse lorsqu'il est déterminé que l'intensité de fluorescence n'est pas à l'intérieur de la gamme prédéterminée ;
le procédé d'amplification d'acide nucléique est **caractérisé par** :
le fait de réinitialiser la valeur de comptage de la chambre défectueuse, d'évacuer la solution de la chambre défectueuse et de remplir la chambre défectueuse avec une nouvelle solution provenant de la partie réservoir (15).
